# EUROPÄISCHE PATENTSCHRIFT

(11) **EP 1 078 026 B1**
(45) Veröffentlichungstag und Bekanntmachung des Hinweises auf die Patenterteilung: **27.07.2005**
(21) Anmeldenummer: 99920863.0
(22) Anmeldetag: 08.04.1999
(51) Int. Cl.: C11B 9/00, A61K 7/46

(54) **VERWENDUNG VON TRICYCLISCHEN ALDEHYDEN ALS RIECHSTOFFE**
UTILIZATION OF TRICYCLIC ALDEHYDES AS ODORIFEROUS AGENTS
UTILISATION D'ALDEHYDES TRICYCLIQUES COMME MATIERES ODORANTES

(30) Priorität: 17.04.1998 DE 19817044
(43) Veröffentlichungstag der Anmeldung: 28.02.2001
(73) Patentinhaber: Kao Corporation, Chuo-Ku Tokyo 103-8210 (JP)
(72) Erfinder: MARKERT, Thomas, D-40789 Monheim (DE)
(74) Vertreter: Wolf, Matthias
(86) Internationale Anmeldenummer: PCT/EP1999/003205
(87) Internationale Veröffentlichungsnummer: WO 1999/054428

(56) Entgegenhaltungen:
- DE-A- 2 623 285
- US-A- 3 985 769
- PATENT ABSTRACTS OF JAPAN vol. 007, no. 095 (C-163), 23. August 1983 (1983-08-23) & JP 58 021638 A (MITSUBISHI YUKA KK), 8. Februar 1983 (1983-02-08) in der Anmeldung erwähnt
- A.SPENCER: "Hydroformylation of cyclic dienes catalysed by acetocarbonylbis(triphenylphosphine)rhodiu m" JOURNAL OF ORGANOMETALLIC CHEMISTRY., Bd. 124, 1977, Seiten 85-91, XP002116124 ELSEVIER-SEQUOIA S.A. LAUSANNE., CH ISSN: 0022-328X in der Anmeldung erwähnt

## Beschreibung

Die Erfindung betrifft die Verwendung spezieller tricycliseher Aldehyde, die erhältlich sind durch partielle oder vollständige Hydroformylierung von - gegebenenfalls (Di)methyl-substituierten - Tricyclo[5.2.1.0^{2,6}]dec(adi)enen als Riechstoffe. Unter "Tricyclo[5.2.1.0^{2,6}]dec(adi)enen" sind dabei sowohl Tricyclo[5.2.1.0^{2,6}]decadiene als auch Tricyclo[5.2.1.0^{2,6}]decene zu verstehen. Unter "(Di)methyl-substituiert" ist zu verstehen, daß das bicyclische System entweder ein oder zwei Methylgruppen als Substituenten tragen kann.

### Stand der Technik

Die Hydroformylierung cyclischer Diene ist literaturbekannt. So beschreibt beispielsweise A. Spencer in Journal of Organometallic Chemistry 1977. 124, Seiten 85 - 91 die Hydroformylierung von unter anderem 1.3- und 1,5-Cyclooctadien in Gegenwart spezieller Rhodiumkatalysatoren. Aus JP 58/21638 ist ein Herstellungsverfahren für Dialdehyde bekannt, bei dem nicht-konjugierte Diolefine in einem nicht mit Wasser mischbaren Lösungsmittel in Gegenwart eines Rhodium-Katalysators mit Wasserstoff und Kohlenmonoxyd umgesetzt werden. Aus US 3 985 769 ist Cyclooctanaldehyd (1a), der gemäß als Rohstoff für die Herstellung davon abgeleiteter Acetale mit Dufteigenschaften beschrieben wird, hinsichtlich seiner geruchlichen Eigenschaften, die als "intensiv grün" beschrieben werden, bekannt.

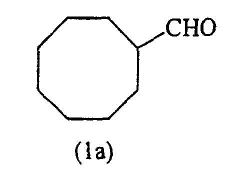

Viele natürliche Riechstoffe stehen gemessen am Bedarf in völlig unzureichender Menge zur Verfügung. Es ist daher klar, daß die Riechstoffindustrie einen ständigen Bedarf an neuen Riechstoffen mit interessanten Duftnoten hat, um die Palette der natürlich verfügbaren Riechstoffe zu ergänzen und die notwendigen Anpassungen an wechselnde modische Geschmacksrichtungen vornehmen sowie den ständig steigenden Bedarf ans geruchsverbessernden für Produkte des täglichen Bedarfs wie Kosmetika und Reinigungsmittel decken zu können.

Darüberhinaus besteht generell ein ständiger Bedarf an synthetischen Riechstoffen, die sich günstig und mit gleichbleibender Qualität herstellen lassen und erwünschte olfaktorische Eigenschafen haben, das heißt angenehme, möglichst naturnahe und qualitativ neuartige Geruchsprofile von ausreichender Intensität besitzen und in der Lage sind, den Duft von kosmetischen und Verbrauchsgütern vorteilhaft zu beeinflussen. Es besteht daher Bedarf an Verbindungen, die charakteristische neue Geruchsprofile bei gleichzeitig hoher Haftfestigkeit, Geruchsintensität und Strahlkraft haben.

### Beschreibung der Erfindung

Überraschenderweise wurde gefunden, daß spezielle tricyclische Aldehyde, die erhältlich sind durch partielle oder vollständige Hydroformylierung von - gegebenfalls (Di)methylsubstituierten - Tricyclo[5.2.1.0^{2,6}]dec(adi)enen über bemerkenswerte Geruchseigenschaften verfügen. Über ihre spezielle Geruchscharakteristik hinaus, die durch eine große Bandbreite mit komplexen Schattierungen gekennzeichnet ist, zeichnen sich die Verbindungen durch gute Haftfestigkeit und Strahlkraft aus. Darüber hinaus eignen sie sich in ausgezeichneter Weise als Duft-Booster. Unter einem Duft-Booster ist dabei eine Substanz zu verstehen, die in der Lage ist, die geruchlichen Eindrücke der Komponenten eines Mehrstoff-Systems, d.h. einer Mischung zweier oder mehrerer Riechstoffe, nachhaltig zu intensivieren.

Gegenstand der Erfindung ist die Verwendung von tricyclischen Aldehyden, die erhältlich sind durch partielle oder vollständige Hydroformylierung von - gegebenfalls (Di)methylsubstituierten - Tricyclo[5.2.1.0^{2,6}]dec(adi)enen, als Riechstoffe. Die Verbindung Tricyclo[5.2.1.0^{2,6}]decanaldehyd (1b) ist dabei ausgenommen. Bei (1b) handelt es sich den nachstehenden Formeln entsprechend um folgende Species:

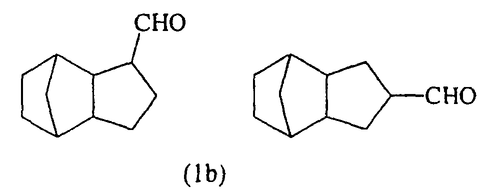

Ein weiterer Gegenstand der Erfindung ist die Verwendung von tricyclischen Aldehyden, die erhältlich sind durch partielle oder vollständige Hydroformylierung von - gegebenfalls (Di)methylsubstituierten - Tricyclo[5.2.1.0^{2,6}]dec(adi)enen, als Duft-Booster, wobei Tricyclo[5.2.1.0^{2,6}]decanaldehyd (1b) ausgenommen ist. Hinsichtlich dieser Verwendung ist 4,8-Di-Formyl-Tricyclo[5.2.1.0^{2,6}]decan besonders bevorzugt.

Die Herstellung der erfindungsgemäß einzusetzenden Aldehyde geschieht vorteilhaft durch Hydroformylierung von - gegebenfalls (Di)methylsubstituierten - Tricyclo[5.2.1.0^{2,6}]dec(adi)enen. Die letztgenannten Verbindungen weisen als gemeinsames Grundgerüst ein tricyclisches System auf. Das Grundgerüst - jedoch ohne die Angabe möglicher Methylgruppen und ohne die Angabe von C=C-Doppelbindungen - wird durch die folgende Formel (2) veranschaulicht:

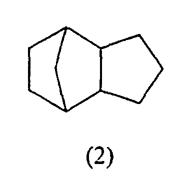

Dieses System (2) enthält optional ein oder zwei Methylgruppen und zwingend ein oder zwei C=C-Doppelbindungen. Sofern zwei C=C-Doppelbindungen vorliegen, gilt, daß diese nicht unmittelbar benachbart sind.

Bei der Hydroformylierung handelt es sich um eine dem Fachmann bekannte Reaktion, die bereits 1938 durch von Roelen entdeckt wurde. Dabei werden Alkene mit Kohlenmonoxyd und Wasserstoff in Aldehyde überführt. Die Reaktion ist auch als Oxo-Synthese bekannt.

Sofern im Rahmen der vorliegenden Erfindung als Ausgangsstoffe - gegebenfalls (Di)methylsubstituierte - Tricyclo[5.2.1.0^{2,6}]dec(adi)ene eingesetzt werden, kann die Hydroformylierung partiell oder vollständig durchgeführt werden. Bei der partiellen Hydroformylierung bleibt eine olefinische Doppelbindung pro Molekül der Ausgangsverbindung erhalten, während nur die andere hydroformyliert wird, bei der vollständigen Hydroformylierung werden zwei CHO-Gruppen in das Molekül eingeführt. Sofern jedoch als Ausgangsstoffe Verbindungen mit nur einer C=C-Doppelbindung pro Molekül eingesetzt werden, ist selbstverständlich lediglich die vollständige Hydroformylierung möglich.

Das Geruchsprofil der erfindungsgemäßen Hydroformylierungsprodukte ist originell und neuartig. In Parfüm-Kompositionen verstärken sie die Harmonie und Ausstrahlung sowie auch die Haftung, wobei die Dosierung unter Berücksichtigung der übrigen Bestandteile der Komposition auf die jeweils angestrebte Duftnote abgestimmt wird.

Daß die erfindungsgemäßen Hydroformylierungsprodukte interessante parfümistische Noten aufweisen, war nicht vorhersehbar und ist eine Bestätigung für die allgemeine Erfahrung, daß die olfaktorischen Eigenschaften bekannter Riechstoffe keinen zwingenden Rückschluß auf die Eigenschaften im weitesten Sinne "strukturanaloger" Verbindungen - im vorliegenden Fall etwa (1a) oder (1b) - oder deren Mischungen untereinander zulassen, weil weder der Mechanismus der Duftwahrnehmung noch der Einfluß der chemischen Struktur auf die Duftwahrnehmung hinreichend erforscht sind, somit also normalerweise nicht vorhergesehen werden kann, ob ein geänderter Aufbau oder spezielle Mischungsverhältnisse bekannter Riechstoffe überhaupt zur Änderung der olfaktorischen Eigenschaften führt und ob diese Änderung positiv oder negativ zu beurteilen ist.

Die erfindungsgemäßen Hydroformylierungsprodukte eigenen sich aufgrund ihrer Geruchsprofile insbesondere auch zur Modifizierung und Verstärkung bekannter Kompositionen. Hervorgehoben werden soll insbesondere ihre außerordentliche Geruchsstärke, die ganz allgemein zur Veredlung von Kompositionen beiträgt.

Bemerkenswert ist femer die Art und Weise, wie die erfindungsgemäßen Hydroformylierungsprodukte die Geruchsnoten einer breiten Palette bekannter Kompositionen abrunden und harmonisieren, ohne jedoch in unangenehmer Weise zu dominieren.

In ganz besonderer Weise eignet sich 4,8-Di-Formyl-Tricyclo[5.2.1.0^{2,6}]decan (3) für die erfindungsgemäße Verwendung als Riechstoff und/oder Duft-Booster.

Dementsprechend ist der Einsatz von 4,8-Di-Formyl-Tricyclo[5.2.1.0^{2,6}]decan beispielsweise in Raumbeduftem besonders vorteilhaft. Darüber hinaus hat sich gezeigt, daß 4,8-Di-Formyl-Tricyclo[5.2.1.0^{2,6}]decan mit besonderen Vorteilen in Reinigungsmitteln zur Verstärkung von Zitrusdüften eingesetzt werden kann.

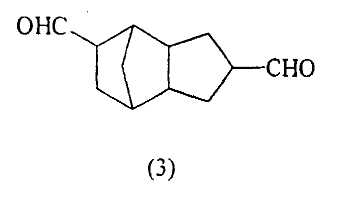

Die einsetzbaren Anteile der erfindungsgemäßen Hydroformylierungsprodukte in Riechstoffkompositionen bewegen sich von 0,001 bis 70 Gew.-%, bezogen auf die gesamte Mischung. Die erfindungsgemäßen Hydroformylierungsprodukte sowie Kompositionen dieser Art können sowohl zur Parfümierung kosmetischer Präparate wie Lotionen, Cremes. Shampoos, Seifen, Salben, Puder, Aerosole, Zahnpasten, Mundwässer. Deodorantien als auch in der Extraitparfiimerie verwendet werden. Ebenso besteht eine Einsatzmöglichkeit zur Parfümierung technischer Produkte sowie Wasch- und Reinigungsmittel, Weichspüler, Textilbehandlungsmittel oder Tabak. Zur Parfümierung dieser verschiedener Produkte werden diesen die Kompositionen in einer olfaktorisch wirksamen Menge, insbesondere in einer Konzentration im Bereich von 0,05 bis 2 Gew.-%, bezogen auf das gesamte Produkt, zudosiert. Diese Werte sollen jedoch keine Grenzwerte darstellen, da der erfahrene Parfümeur auch mit geringeren Konzentrationen Effekte erzielen oder aber mit noch höheren Dosierungen neuartige Komplexe aufbauen kann.

Die vorliegenden Beispiele sollen den Gegenstand der Erfindung erläutern und sind nicht einschränkend aufzufassen.

### Beispiele

### 1. Herstellung von 4,8-Di-Formyl-Tricyclo[5.2.1.0^{2,6}]decan (3)

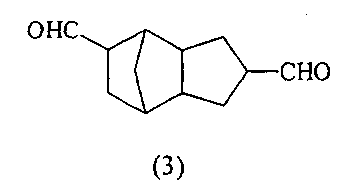

2 mol (264,4 g) Dicyclopentadien (Hersteller: Fa. Acros; gaschromatographisch bestimmte Reinheit: 95%) wurden mit 1,7 mmol (1,175 g) eines Rhodium-Katalysators der Formel Rh(CO)Cl(PPh₃)₂ und 19 mmol (5 g) Triphenylphosphin zusammen in einem Autoklaven vorgelegt und ohne Lösungsmittel unter starker Durchmischung mit einer 1 : 1 Mischung Wasserstoff/Kohlenmonoxid bei einem Druck von 60 kg/cm² bei 100 °C für 5 Stunden zur Reaktion gebracht. Zur Aufarbeitung wurde der Reaktorinhalt filtriert und über eine 20 cm Vigreux-Kolonne destilliert.

Die Fraktionierung in Edukt, Monoaldehyd und Dialdehyd erfolgte an einer Drehbandkolonne. Es wurden 118,6 g eines Gemisches von 2,8- 3.8- und 4,8-Di-Formyl-Tricyclo[5.2.1.0^{2,6}]decan erhalten (Siedepunkt: 65-75 °C/0,07 mbar).

Geruchsbeschreibung: Fichten-Note

## Patentansprüche

1. Verwendung von tricyclischen Aldehyden, die erhältlich sind durch partielle oder vollständige Hydroformylierung von - gegebenfalls (Di)methylsubstituierten - Tricyclo[5.2.1.0^{2,6}]dec(adi)enen als Riechstoffe, wobei Tricyclo[5.2.1.0^{2,6}]decanaldehyd ausgenommen ist.

2. Verwendung von 4,8-Di-Formyl-Tricyclo[5.2.1.0^{2,6}]decan als Riechstoff.

3. Verwendung von tricyclischen Aldehyden, die erhältlich sind durch partielle oder vollständige Hydroformylierung von - gegebenfalls (Di)methylsubstituierten - Tricyclo[5.2.1.0^{2,6}]dec(adi)enen, als Duft-Booster, wobei Tricyclo[5.2.1.0^{2,6}]decanaldehyd ausgenommen ist.

4. Verwendung von 4,8-Di-Formyl-Tricyclo[5.2.1.0^{2,6}]decan als Duft-Booster.

5. Venvendung von tricyclischen Aldehyden, die erhältlich sind durch partielle oder vollständige Hydroformylierung von - gegebenfalls (Di)methylsubstituierten - Tricyclo[5.2.1.0^{2,6}]dec(adi)enen, in kosmetischen Präparaten, technischen Produkten oder der alkoholischen Parfümerie, wobei Tricyclo[5.2.1.0^{2,6}]decanaldehyd ausgenommen ist.

6. Verwendung von 4,8-Di-Formyl-Tricyclo[5.2.1.0^{2,6}]decan in kosmetischen Präparaten, technischen Produkten oder der alkoholischen Parfümerie.

## Claims

1. Use of tricyclic aldehydes which can be obtained by partial or complete hydroformylation of - optionally (di)methyl-substituted - tricyclo[5.2.1.0^{2.6}]dec(adi)enes as perfumes, wherein tricyclo[5.2.1.0^{2.6}]decane aldehyde is excepted.

2. Use of 4,8-di-formyl-tricyclo[5.2.1.0^{2.6}]decane as perfume.

3. Use of tricyclic aldehydes which can be obtained by partial or complete hydroformylation of - optionally (di)methyl-substituted - tricyclo[5.2.1.0^{2.6}]dec(adi)enes as fragrance boosters, wherein tricyclo[5.2.1.0^{2.6}]decane aldehyde is excepted.

4. Use of 4,8-di-formyl-tricyclo[5.2.1.0^{2.6}]decane as fragrance booster.

5. Use of tricyclic aldehydes which can be obtained by partial or complete hydroformylation of - optionally (di)methyl-substituted - tricyclo[5.2.1.0^{2.6}]dec(adi)enes in cosmetic preparations, industrial products or alcoholic perfumery, wherein tricyclo[5.2.1.0^{2.6}]decane aldehyde is excepted.

6. Use of 4,8-di-formyl-tricyclo[5.2.1.0^{2.6}]decane in cosmetic preparations, industrial products or alcoholic perfumery.

## Revendications

1. Utilisation d'aldéhydes tricycliques que l'on peut obtenir par hydroformylation partielle ou complète de tricyclo[5.2.1.0^{2,6}]déc(adi)ènes, éventuellement substitués par un groupe (di)méthyle, en tant que substance odorante, à l'exception de l'aldéhyde de tricyclo[5.2.1.0^{2,6}]décane.

2. Utilisation de 4,8-di-formyl-tricyclo[5.2.1.0^{2,6}]décane, en tant que substance odorante.

3. Utilisation d'aldéhydes tricycliques que l'on peut obtenir par hydroformylation partielle ou complète de tricyclo[5.2.1.0^{2,6}]déc(adi)ènes - éventuellement substitués par un groupe (di)méthyle, en tant que booster de parfum, à l'exception de l'aldéhyde de tricyclo[5.2.1.0^{2,6}]décane.

4. Utilisation de 4,8-di-formyl-tricyclo[5.2.1.0^{2,6}]décane, en tant que booster de parfum.

5. Utilisation d'aldéhydes tricycliques que l'on peut obtenir par hydroformylation partielle ou complète de tricyclo[5.2.1.0^{2,6}]déc(adi)ènes - éventuellement substitués par un groupe (di)méthyle - dans des préparations cosmétiques, des produits industriels ou les parfums alcoolisés, à l'exception de l'aldéhyde de tricyclo[5.2.1.0^{2,6}]décane.

6. Utilisation de 4,8-di-formyl-tricyclo[5.2.1.0^{2,6}]décane dans des préparations cosmétiques, des produits industriels ou les parfums alcoolisés.
